# EUROPEAN PATENT APPLICATION

(11) **EP 2 364 697 A1**
(43) Date of publication of application: **14.09.2011**
(21) Application number: 10425067.5
(22) Date of filing: 12.03.2010
(51) Int. Cl.: A61K 31/122, A61K 31/353, A61K 31/355, A61K 31/385, A61K 31/4415, A61P 39/06

(54) **Antioxidant composition for reducing oxidative stress ascribable to the treatment with hormonal contraceptive drugs**

(71) Applicant: Cor.Con. International S.r.L., 43124 Parma (IT)
(72) Inventor: Cornelli, Umberto, 20129 Milano (IT)
(74) Representative: Cattaneo, Elisabetta

(57) **Abstract**

An antioxidant composition is described for reducing oxidative stress in subjects undergoing hormonal contraceptive drug treatment. In particular, said composition has proved to be particularly suitable because, as well as being surprisingly effective, it is extremely well-tolerated by the body as it contains natural components such as catechins from green tea, selenium yeast, alpha-lipoic acid, and coenzyme Q10.

## Description

### FIELD OF THE INVENTION

The present invention concerns an antioxidant composition for reducing oxidative stress in subjects undergoing treatment with hormonal contraceptive drugs. This composition has proved to be particularly suitable because, as well as being surprisingly effective, it is extremely well-tolerated by the body.

### STATE OF THE ART

In the 50's it was discovered that progesterone inhibits ovulation and that the problem of controlling pregnancies presented highly complex and delicate aspects, in that side-effects were observed over time such as thrombotic phenomena, the development of certain tumour types (i.e. of the breast), as well as, conversely, a reduction in other tumour types (i.e. colon, rectal, ovarian). A long debated and finally proven factor is the presence of oxidative stress (OS) when contraceptive treatment (CT) is used [1,2]. OS can actually promote the onset of cardiovascular diseases and thrombotic episodes and is also present when low estrogen-progestin dosages are used, such as to appear to be closely linked to the contraceptive treatment itself.

Although the reasons behind these events have not yet been fully clarified, the principle remains of achieving the best compromise between low dosage and effectiveness.

In effect, attempts to optimize the benefit/risk relationship have ranged from high progestin dosages to combinations of estrogens with progestins of various types. In this sense, transdermal release systems, interuterine release systems (IUD, inter uterine device) and systems with subcutaneous or intramuscular depots are known. Generally, the various types of contraceptives currently available are prescribed on the basis of health status and lifestyle habits (such as smoking). However, even in the case of healthy women, an increase in oxidation phenomena has been noted during the menstrual cycle which, though not reaching levels such as to be classified as OS, nevertheless indicate an increase in oxidative phenomena at the ovum maturation phase (estrogen phase) and the subsequent phase of its possible implantation (progesterone phase).

In order to understand the implications underlying the onset of OS, the hormonal aspects of the menstrual cycle are closely examined.

### Hormonal aspects related to the normal menstrual cycle

The steroid hormones related to a woman's reproductive function are regulated by a central mechanism located in the arcuate nucleus of the hypothalamus and the anterior lobe of the pituitary gland, the neurons of which are in connection with the adrenal glands, the uterus and ovaries.

In brief [3], the neurons of the arcuate nucleus release GnRH (gonadotropin-releasing hormone) every hour in a timed manner under noradrenergic stimulus; in contrast, the opioid, dopaminergic and gabaergic neurons maintain said neurons in an inhibitory state.

It is evident, therefore, that it is the balance between these "drivers" which creates the central vectorial stimulus in terms of frequency and amplitude (one peak/hour with amplitude around 2 mlU/ml of LH).

GnRHs reach the anterior pituitary gland via the so-called portal circulation (in a spiral form) wrapped around the pituitary gland and stimulate it to release bursts of FSH (follicle-stimulating hormone) and LH (luteinizing hormone), hence in relation with GnRH release; these FSHs and LHs regulate ovarian production of estradiol (E₂) and progesterone (Pg).

E₂ is the hormone of the so-called proliferative phase; it enables maturation of the ovarian follicle and stimulates endometrial cell proliferation and differentiation. Both the mucosal cells and those of the endometrial vessels increase in number (and in volume); moreover, E₂ induces the production of Pg receptors in cells, so that they become more reactive to its presence when the corpus luteum begins to produce it.

Pg is the hormone of the secretory phase, i.e. the phase that prepares to receive the ovum, and derives from the corpus luteum which forms from the stroma of the empty ovarian follicle. Its action limits the proliferative effect and more precisely stimulates vasal differentiation and production of secretions more consistent with implantation of the ovum (blastocyst). In this phase, progesterone also stimulates neurons at the central level (opioid, dopaminergic and gabaergic), which inhibit GnRH release.

Both E₂ and Pg, concurrently with FSH, modify LH production by the pituitary; this modification is achieved by reducing the peak frequency of LH (one peak/3 hours instead of 1 peak/hour) and increasing its amplitude (from 2 mlU/ml to 3-4 mlU/ml of LH).

Thus latter aspect operates to reduce LH secretion should the ovum not have implanted, so that E₂ and Pg levels decline and so ending the cycle with the menstrual flow.

However, should the ovum instead implant, chorionic gonadotropins (hGC) produced by the trophoblast and then by the placenta which will form, stimulate the corpus luteum to continue producing E₂ and Pg which function in maintaining the condition of the uterus for pregnancy. Once formed, the placenta will be self-sufficient in producing both estrogens.

These estrogens also perform auxiliary, but important, activities for the purpose of reproduction; in the fallopian tubes, E₂ stimulates proliferation and differentiation while Pg reduces it; E₂ increases myometrial contractility while Pg reduces it; E₂ increases the quantity and fluidity of cervical mucus (to facilitate passage of spermatozoa) while Pg reduces it, makes it more viscous and alters its consistency. One of the most debated problems is however the action of E₂ and Pg on oxidative stress. This dualism, whereby one has an action opposite to the other, does not seem to be practicable, since both have a stimulating action on cellular energy use and metabolic induction, though for different tasks. Therefore, in purely theoretical terms, both could be pro-oxidants, but the oxidative balance depends on how much the stimuli of one or the other allow production of endogenous antioxidants. Therefore, the state of the art on this particular topic is yet to be clarified and some salient aspects will be discussed forthwith.

### Condition of OS during the normal menstrual cycle

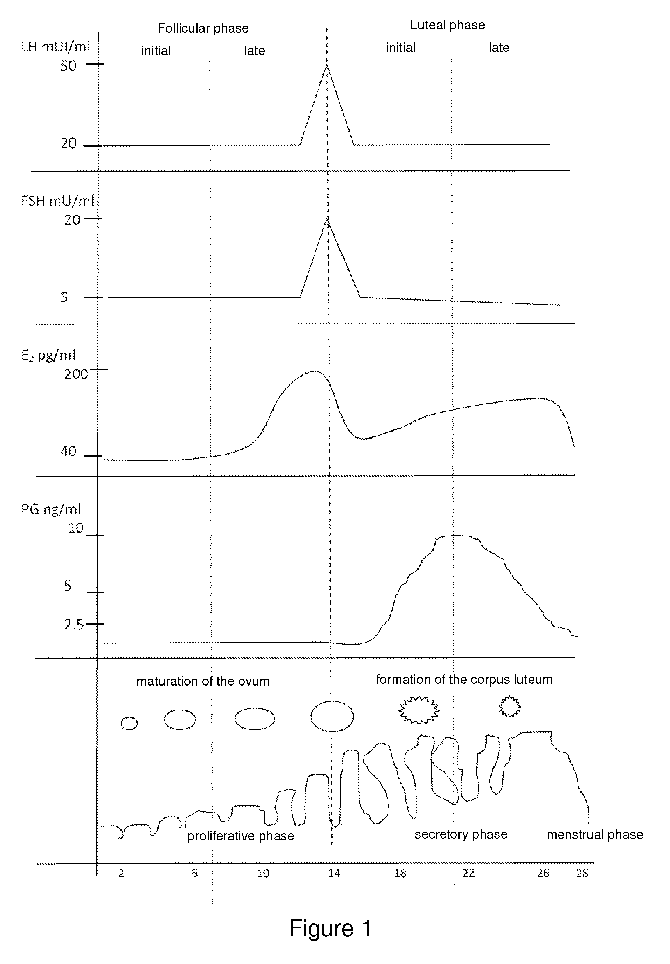

As shown in Figure 1, both the follicular and luteal cycles are schematically subdivided into an early phase and a late phase. These enable the progression of the condition of OS described by some authors [4] to be differentiated in diagrammatic form.

By basing the analysis on local concentrations (endometrial tissue) of GSH (reduced glutathione), GSSG (oxidized glutathione), GSHpx (glutathione peroxidase enzyme) and lastly MDA (malonyl dialdehyde), changes in oxidative conditions could be assessed. Said conditions were then correlated with levels of hormones (estradiol, progesterone), LH and FSH.

These data show that that the peak in OS appears at the mid-cycle phases (late follicular and early luteal), i.e. at maturation of the ovum and possible ovum implantation. This phase is manifested by: a) an increase in production of GSHpx; b) a reduction in GSH; c) an increase in GSSG; d) the substantial stability of MDA. This means that as OS increases, there is a corresponding compensation such that the MDA concentration (representing lipid peroxidation) remains constant; in other words the system is kept in equilibrium.

The OS peak corresponds to the peak of estrogens and LH, while the progesterone peak appears to correspond to the OS recovery stage.

In this regard, it should be noted that GSHpx is the enzyme system that utilizes GSH as substrate, enabling hydroperoxides of varying types to be reduced (H₂O₂, or of lipid nature such as ROOH etc.) forming H₂O

There are two types of GSHpxs, namely GSHpx 1 which is found in the cytoplasm of all cells including erythrocytes, and GSHpx 3 which is found in extracellular fluids, including plasma. The two enzyme types, though both formed from four identical sub-units each containing a seleno-cysteine (SeCys), in reality are different. In this respect, polyclonal antibodies against GSHpx 1 do not react with GSHpx 3. The GSHx3 found in plasma is synthesized in the most part by the proximal tubule cells of the kidney. However, other cells are also able to synthesize it (liver, lung, heart, breast, intestine, brain, muscles) and secrete it [5]. The GSH-catalyzed reaction in relation to hydroperoxides is the following:

ROOH + 2 GSH = ROH +H₂O + GSSG

Therefore, an increase in GSHpx corresponds in functional terms to an increase in GSH oxidation; hence, in biological systems, an increase thereof corresponds physiologically to a reduction in GSH with a consequent increase in GSSG. All of this signifies OS; therefore, it is mistaken to consider GSHpxs (of any type, 1 or 3) to be an index of antioxidant capacity, instead they must be considered as an expression of an increased need for using GSH to counteract OS.

However, if a clinical condition is characterized by reduced levels of GSHpx, this means that using GSH in order to counteract OS will be more difficult.

This occurs in the menopause, for example, both in the endometrium and plasma, though it also seems to be related to advancing age (a typical condition of the menopause), and hence to a reduced efficiency of the antioxidant system.

When the GSHpx, SOD (superoxide dismutase) and CAT (catalase) systems are analyzed in plasma during the menstrual cycle [6], the levels of erythrocyte GSHpx are seen to increase in the late follicular and early luteal phase relative to the other phases, while the SOD and CAT levels remain essentially unchanged (see Table I below). Analysis of the correlations with E₂ levels indicates that GSHpx levels are time-correlated (in extreme synthesis, an increase in one is simultaneous with an increase in the other).

Levels of FSH, LH, progesterone, testosterone and androstenedione do not show patterns that are time-correlated with those of erythrocytic GSHpx.

From that stated and demonstrated, the isolatable action of E₂ is not antioxidative but, rather, precisely the opposite [7] and corresponds to the cellular activation needed for increased energy requirement in the secretory phase.

If the ovum has not been fertilized, the luteal phase is also characterized by an increased cellular activity and tissue proliferation, which precedes the end of the cycle and menstrual flow, returning the uterine mucosa back to the conditions at the start of the cycle.

This phase is expressed differently in terms of final activity (changes in mucus viscosity, increase in vascular component etc.) but in terms of oxidation remains practically the same, it being a proliferative phase.

This phase is dominated by production of Pg which is combined to that of E₂, and as such is the phase with the highest expression of estrogenic-progestogenic steroids, after the phase of ovum maturation.

**Table I. Changes in erythrocyte levels of enzymes pertaining to oxidation**

| Variables | Phases of the cycle | | | |
|---|---|---|---|---|
| | Initial follicular | Late follicular | Initial luteal | Late luteal |
| GSH (IU/g Hb) | 15.2 ± 0.38 | 16.7 ± 0.23 | 16.3 ± 0.22 | 14.1 ± 0.31 |
| SOD (IU/100 µg Hb) | 1.3 ± 0.04 | 1.3 ± 0.02 | 1.2 ±0.03 | 1.3 ± 0.02 |
| CAT (IU/g Hb) | 18.6 ± 3.24 | 20.6 ± 2.28 | 19.6 ± 2.56 | 18.2 ± 3.12 |

A further characteristic of this part of the cycle is the considerable production of the NO^{●} radical. NO^{●} is an important mediator for vasodilation, for relaxation of the myometrium, anti-aggregation action and angiogenic stimulus which is carried out by VEGF (vascular endothelial growth factor) stimulating NO synthase [8], meaning that NO^{●} seeks to maintain itself through autoinduction of NO synthase. Although this latter occurs in all the menstrual phases, both as eNOS (endothelial) or iNOS (inducible), it increases substantially on progestogenic stimulation [9]. Concurrently with this increase, there is also a local increase in SOD. SOD, which dismutes O₂^{●} (superoxide), prevents the reaction NO^{●} + O₂^{●} from forming the ONOO⁻ ion (peroxynitrite) which, as well as having a high oxidizing power, removes NO^{●} and restricts its availability. But all said available NO^{●} reacts with O₂^{●} (the reaction between the two is by far the most rapid in the body (1 x 10⁻⁹ seconds) and at the end produces OS.

Vascular patency of the endometrium and its regular blood supply are also maintained by a specific peptide hormone, namely relaxin (RLX).

RLX is a peptide with a MW of 6 kDa, consisting of two peptide chains, stabilized both internally and with each other by S-S bridges [10]. Actually, this is a family of hormones (H₁, H₂, H₃) of which the circulating form H₂ is the most represented. RLX is mainly secreted by the corpus luteum and strategically prevents changes in endometrial flow to avoid ischemia and reperfusion phenomena during the proliferative phase of the cycle and then during a possible pregnancy, which could trigger reactive processes with production of ROS and chemotaxis.

RLX action occurs by upregulating iNOS and eNOS and angiogenesis, particularly, but not only, in the endometrium; this action occurs both on the arterial and the venous vessels (hence also on vessels without smooth muscle). The concentrations at which RLX is active are at nano-molar levels, hence achievable during the endometrial phase proper of ovum implantation and pregnancy. Also typical of RLX is its angiogenic action, carried out by the release of VEGF and also bFGF (basic fibroblast growth factor).

An important aspect of RLX's action is to inhibit adhesion of inflammatory white blood cells to the endothelium and platelet aggregation. This activity, together with upregulation of NO^{●} production, prevents the generation of ROS and chemotactic attraction, typical of the ischemia/reperfusion condition.

On the whole, therefore, RLX substantially influences blood perfusion and supply in the endometrium, maintaining ideal flow conditions therein and performing typical anti-inflammatory and anti-thrombotic actions.

### Action of estrogens on the oxidation of lipoproteins

A final factor to be considered is the effect of estrogens on lipoprotein oxidation. Most experiments carried out in vitro or ex vivo have demonstrated the antioxidant capacity of E₂, and that its effect on lipid peroxidation is similar to that of α-tocopherol or β-carotene, but in a clearer manner than its metabolites estradiol and estrone.

Actually, most of the experiments performed [11-12] were carried out by using much higher than physiological concentrations, at least in the order of 1-10 nmol/ml, corresponding to 273 and 2730 ng/ml respectively. These concentrations are barely reached when estrogen peaks at mid-cycle and only for limited time periods; for this reason they can only difficultly claim a protective antioxidant action for LDL [13].

However, in an experiment conducted in menopausal women, administration of E₂ intra-arterially or in the form of a transdermal patch (for 3 weeks) has clearly shown a delay in LDL oxidation (increased lag time or time of oxidation by Cu²⁺) of 11 % and 16% respectively, with a return to baseline conditions on discontinuation of the treatment [12]. Strong doubts remain, however, that E₂ levels obtained by means of the two administration routes were actually physiological, in that they were determined on one point only; one only has to consider the blood peak obtained with intra-arterial administration before its distribution stabilizes. Other than this, no differences in protective activity of LDL were noted between the two administration types despite the considerable differences in levels (from 4 to 7 times higher with the patch).

From the various studies carried out, it follows that in order to observe an antioxidant effect (notwithstanding the problem of levels) E₂ should be in contact with plasma, since if it is simply added to LDL no activity is found [11 ]. This phenomenon has been interpreted as E₂ needing to be activated by a blood component, determined as being LCAT (lecithin-cholesterol acyltransferase) which esterifies E₂ with a fatty acid; only under these conditions can it exert a protective effect towards lipoproteins. The esterification appears to takes place on the C17 carbon, leaving free the hydroxyl group in C3; this ester appears to be inserted in the lipoprotein membrane with the hydrophilic part turned towards the outer surface and the lipophilic part within the membrane [14]. A similar configuration could in theory protect the membrane from oxidation, accordingly by oxidizing the E₂ ester, but whether this can suffice in protecting a LDL remains unknown.

The problem was hence assessed from the stoichiometric viewpoint.

The levels of E₂-esters in pre-menopausal women are scarcely measurable and the highest levels are found in follicular fluid at concentrations of about 0.1 µmol/l [15]. All this means that the ratio between the lipoproteins (LDL, HDL, VLDL) is very high since only LDLs are present in blood to the extent of at least 0.6 µmol/l. (corresponding to 1.6 g/I for a mw of 2.6 x 10⁶). Therefore the presence of E₂-esters in lipoproteins is very small (a molecule of E₂-ester per thousands of lipoproteins), and hence not compatible with a direct antioxidant action; the possible mechanism of LDL protection may be of enzymatic type only (activation of the antioxidant enzymes present in lipoproteins, such as paraoxonase, apoA, apoJ, GSHpx), but is currently unknown.

With regard to the types of E₂-esters, those isolated from follicular fluid are predominantly unsaturated i.e. 96% (linoleates, arachidonates, palmitates) and only 4% are saturated (stearates) [15].

E₂-esters are hypothesized to be a means of keeping E₂ available as a reserve, as their presence is relatively consistent in omental and subcutaneous lipids (about 1 pmol/g of tissue) in pre-menopausal women, while they tend to fall substantially during menopause [16].

One of the considerations to be made regarding their presence on lipoproteins, is that in this manner, in contrast to free estrogens, they cannot be eliminated via the kidneys and are released into cells through the LDL receptor route. Because of their liposolubility, free estrogens can easily cross the cell membrane in a less controllable manner.

Therefore, on the whole, the strategic significance of E₂-esters is undoubtedly not to act as direct antioxidants but, if anything, to be indirect antioxidants and to modulate E₂ release to create a reserve to be activated as required. The mechanism for forming the esters of steroidal hormones and their scanty presence in the circulation in a nevertheless protected form (lipoproteins), without binding to albumin/globulin nor to SBP (steroid binding proteins) or CBG (corticosteroid binding globulins), is evidence of the need for using them as steroid reserves ready to be utilized if required.

The E₂ are easily oxidizable, whereas the E₂-esters are less so.

The presence of MPO (myeloperoxidase) and H₂O₂ (this latter also secreted by the endothelial cells) induces oxidation of E₂ which behaves as a propagator of oxidation as well as inducing MPO production by the neutrophils [13].

However, the oxidative aspect of E₂ is very particular.

Its behaviour seems similar to that occurring during physical activity [17]; that is to say the LDLs are oxidized and taken up more easily from the circulation by hepatic receptors and kept under control and partially "subdued" by the antioxidant system in muscle. However, if the antioxidant system is insufficient and their levels increase, they undergo vascular uptake (subendothelial) which triggers the process of inflammatory arteriosclerosis. They would in fact be subject to MPO activity in the subendothelium.

This accounts for the reduced cholesterol and LDL in premenopausal women, interpreted as a greater hepatic LDL receptor efficiency and not as an increased uptake of LDL, due to their being in partially oxidized conditions (it should be noted that there is a minimum oxidation limit available above which the macrophage reaction is triggered).

This nearly balanced oxidative equilibrium can be described as balanceable under physiological conditions of normal estrogen-progesterone secretion (hence during a normal menstrual cycle) but can change towards pro-oxidation when contraceptive (CT) therapy is used.

The phenomenon is understandable, since any oral estrogen-progestin administration produces a blood peak of both (depending on the CT type), being not of physiological size, and hence the "slight and controllable" oxidation becomes OS.

The fact that this OS is generated by an increase in the amount of oxidized LDL, or an increase in blood Cu levels, or the up-regulation of iNOs [1] or by a combination thereof has now been demonstrated [1, 2]. The problem which arises is that of controlling OS, but in order to achieve this it is first necessary to realize what is happening in terms of OS in normal conditions so as to take action to reduce stress when taking contraceptive therapy.

The need was therefore felt to find an effective remedy, well-tolerated by the body and able to reduce oxidative stress ascribable to the treatment with hormonal contraceptive drugs. It is therefore an object of the present invention to provide such a remedy.

### Summary of the invention

This object was achieved by means of the antioxidant composition as claimed in claim 1, comprising selenium yeast, vitamin B6, alpha-lipoic acid, coenzyme Q10, beta-carotene and catechin.

In another aspect, the present invention concerns the use of said composition for reducing oxidative stress ascribable to the treatment with hormonal contraceptive drugs.

In a further aspect, the invention concerns a kit for the administration of said composition.

As will be evident from the following detailed description, the composition of the invention has surprisingly enabled oxidative stress to be reduced in individuals undergoing treatment with hormonal contraceptive drugs, by a suitable combination of antioxidant components showing high tolerance by the body.

### BRIEF DESCRIPTION OF THE DRAWING

The characteristics and advantages of the present invention will be evident from the following detailed description, from the working examples given for illustrative and non-limiting purposes, and from the annexed Figure 1, wherein the significant events of the menstrual cycle are diagrammatically presented.

### DETAILED DESCRIPTION OF THE INVENTION

The present invention therefore relates to an antioxidant composition comprising selenium yeast, vitamin B6, alpha-lipoic acid, coenzyme Q10, beta-carotene and catechins. In fact, it has been observed that the combination of these components surprisingly and advantageously enables the oxidative stress associated with hormonal contraceptive drug consumption to be reduced. This formulation advantageously contains all types of antioxidants, i.e. systemic (selenium), cytoplasmic (vitamin B6), mitochondrial (alpha-lipoic acid, coenzyme Q10), membrane (beta-carotene), circulating (catechins).

Catechins are antioxidative plant-derived metabolites belonging to the flavonoid group. They are found in a number of plant species, but the most important source in man's diet is the various teas deriving from the tea plant, Camellia sinensis. The dried tea leaf actually contains around 25% by weight of catechins, although the total content can vary significantly according to the type of plant, the site of growth, the variations in light, season and altitude. In particular, catechins are present in all types of tea, including white tea, green tea, black tea and oolong, but are also present in chocolate, fruit, vegetables, wine and many other plant species. In accordance with a preferred embodiment of the invention, said catechins derive from extract of green tea.

Preferably, said antioxidant composition comprises 8-16% by weight of selenium yeast, 0.5-5% by weight of vitamin B6, 1-10% by weight of alpha-lipoic acid, 1-10% by weight of coenzyme Q10, 0.05-0.5% by weight of beta-carotene and 40-60% by weight of catechin extract. This preferred combination exerts a complete and synergistic antioxidant action. In particular, in the stated proportions the prevailing antioxidant action is considered to be ascribable to circulating antioxidants.

More preferably, said antioxidant composition comprises 10-14% by weight of selenium yeast, 1-3% by weight of vitamin B6, 3.5-6% by weight of alpha-lipoic acid, 3.5-6% by weight of coenzyme Q10, 0.1-0.3% by weight of beta-carotene and 42-51% by weight of catechins. In this case, the antioxidant combination in these ranges enable a wider expression of the "non-circulating" antioxidant categories and hence a conveniently more prolonged action over time.

Even more preferably, said antioxidant composition comprises 12-13% by weight of selenium yeast, 1.1-2% by weight of vitamin B6, 4-5.5% by weight of alpha-lipoic acid, 4-5.5% by weight of coenzyme Q10, 0.2-0.3% by weight of beta-carotene and 45-48% by weight of catechins. In this case, as well as a longer duration in time there is also a more potent action provided by an ideal ratio between systemic antioxidants, cellular antioxidants, mitochondrial antioxidants, membrane antioxidants and circulating antioxidants such as catechins.

In particular, said catechins are epigallocatechin gallate (EGCG), epigallocatechin (EGC), epicatechin gallate (ECG) and epicatechin (EC). In accordance with a further preferred embodiment, at least 35% by weight on the weight of the catechins is EGCG. Catechins, besides exhibiting good antioxidant activity, have also demonstrated good bioavailability enabling, particularly EGCG, to reach tissue levels of around 8-10% of blood levels in the principal organs (heart, brain, liver, uterus etc.)

According to another preferred embodiment, said vitamin B6 is pyridoxine or a salt thereof. More preferably, it is pyridoxine hydrochloride.

In another aspect, the invention concerns an antioxidant composition for use as a medicament. In particular, said antioxidant composition is used for reducing the oxidative stress ascribable to the treatment with hormonal contraceptive drugs. As will in fact be seen from the working examples to follow, the composition of the invention has shown a surprising synergistic effect deriving from the opportune combination of the various components, which separately have not conversely demonstrated any effectiveness.

Preferably, said antioxidant composition is to be administered orally.

Preferably, the antioxidant composition of the invention is in a single unit dose form comprising 15.8-31.5 mg of selenium yeast, 1-9.5 mg of vitamin B6, 2-19 mg of alpha-lipoic acid, 2-19 mg of coenzyme Q10, 0.1-0.95 mg of beta-carotene and 80-110 mg of catechins.

More preferably, said unit dose comprises 20-27.5 mg of selenium yeast, 2-5.5 mg of vitamin B6, 7-11.8 mg of alpha-lipoic acid, 7-11.8 mg of coenzyme Q10, 0.2-0.59 mg of beta-carotene and 85-95 mg of catechins.

In accordance with a preferred embodiment the unit dose comprises 24 mg of selenium yeast, 2.44 mg of vitamin B6, 10 mg of alpha-lipoic acid, 10 mg of coenzyme Q10, 0.5 mg of beta-carotene and 90 mg of catechins, wherein about 40% is EGCG.

In a further aspect, the present invention concerns a kit comprising:
i) at least one antioxidant composition comprising selenium yeast, vitamin B6, alpha-lipoic acid, coenzyme Q10 and beta-carotene; and
ii) at least one aqueous solution comprising catechins,
for the separate, sequential or simultaneous administration of the antioxidant composition as above described.

Preferably, in said kit, said antioxidant composition is in the form of a powder in order to improve its conservation over time.

It should be understood that all aspects identified as preferred and advantageous for the antioxidant composition as above described are also to be considered accordingly preferred and advantageous for the kit of the present invention.

In accordance with a preferred embodiment, the powder is taken up with said solution at the time of administration. Furthermore, said solution is preferably decaffeinated green tea.

Working examples of the present invention herein below provided for illustrative and non-limiting purposes.

### EXAMPLES

### Example 1.

*Determination of the antioxidant power of the compositions of the invention by the*

### BAP test (biological antioxidant potential)

10 eumenorrheic women volunteers were selected. The general characteristics of the volunteers are given below:

| Characteristics | Mean values ± SD* |
|---|---|
| Age (years) | 28.2 ± 3.91 |
| Weight (kg) | 61.4 ± 3.78 |
| Height (m) | 1.66 ± 0.04 |
| Body Mass Index (BMI kg/m²) | 22.5 ± 2.01 |

| | |
|---|---|
| * SD = standard deviation | |

The healthy volunteers were required to consume three different compositions (1 A, 1 B, 1 C) of different dosages, with the aim of determining the most effective combination in terms of activity and duration over time.

The three compositions were analyzed, the contents of which are given in Table 1.

**Table 1. Compositions tested for activity by the BAP test**

| | Amount | | Compositions | |
|---|---|---|---|---|
| | | 1A | 1B | 1C |
| Catechins | mg | 99.5 | 93.5 | 90 |
| Se yeast | mg | 18 | 21 | 24 |
| Vitamin B6 | mg | 5 | 4 | 2,4 |
| Alpha-lipoic acid | mg | 4 | 8 | 10 |
| Coenzyme Q10 | mg | 4 | 8 | 10 |
| Beta-carotene | mg | 0.2 | 0.3 | 0.5 |

The catechins were supplied in the form of a green tea extract, wherein said catechins were about 60% by weight.

In the three compositions, from 1A to 1C, the amount of catechins and pyridoxine (in hydrochloride form) was progressively reduced and the amounts of alpha-lipoic acid, beta-carotene, selenium and coenzyme 10 were simultaneously increased. In other words, in the various compositions from A to C, the amount of soluble antioxidants was reduced and that of liposoluble antioxidants was increased.

All three compositions were administered to the volunteers over three successive days at the end of their cycle starting from the third day after the start of the menstrual flow.

During this period no other therapies were permitted. The experiment was performed in the morning while fasting from the previous evening and only water consumption was allowed during the period from 7.00 to 13.00.

Administration of the compositions was always undertaken in the order A, B, C. The BAP test (distributed by Diacron Srl, Grosseto, Italy) was performed by using finger prick blood collected into heparinized microtubes (0.15-0.2 ml of blood); four collections were taken: the basal, and at 1 hour, 3 hours and 5 hours (respectively T1, T2, T3) after administration of each composition.

The differences between the values were calculated on the basis of the t-test and analysis of variance for orthogonal contrasts (ANOVA). The results are given in Table 2.

**Table 2. BAP values at the various observation times following treatment with three different compositions of the invention. Mean values ± SD (standard deviation).**

| Times | Compositions | | | ANOVA |
|---|---|---|---|---|
| | 1A | 1B | 1C | |
| Basal | 1965 ± 250.4 | 1994 ± 178.2 | 1977 ± 172.8 | A=B=C p > 0.05 |
| T1 (after 1 h) | 2415 ± 360.1^{a} | 2474 ± 312.7^{a} | 2605 ± 234.8^{a} | C>A=B p < 0.05 |
| T2 (after 3 h) | 2238 ±266.5^{a} | 2453 ± 279.6^{a} | 2448 ± 207.3^{a} | B=C>A p < 0.05 |
| T3 (after 5 h) | 1915 ± 224.6 | 2223 ± 256.6^{a} | 2270 ±190.7^{a} | B=C>A p < 0.05 |

| | | | | |
|---|---|---|---|---|
| ^{a}= t-test for interdependent data; TₙVs Basal p< 0.05 | | | | |

It could be seen from the data that all three compositions advantageously increased the antioxidant power of plasma significantly; the compositions 1 B and 1 C additionally gave a more prolonged action than composition 1 A; after 1 hour composition 1 C unexpectedly showed a significantly better action than the others. From observations, it was thus shown that all the compositions had a considerable antioxidant power, and were hence bioavailable, with composition 1 C having a marked efficacy and showing a more substantial and prolonged action over time.

### Example 2.

### Determination of oxidative stress (OS) during the normal menstrual cycle

For this test, 20 eumenorrheic women volunteers were selected. They were apparently healthy volunteers, were not under any therapy and were for the most part nulliparous (14 out of 20). Use of any therapy with food supplements was considered an exclusion criterion.

The OS during the menstrual cycle (Cycle A) was analyzed using the d-ROMs test [18], which enabled evaluation of hydroperoxides in the blood (plasma or serum), detectable after a finger prick, in blood (0.15-0.2 ml) collected in heparinized microcuvettes. The test was carried out starting from the first day after the end of menstrual flow and then on the third day and subsequently every three days (indicated as t₁, t₃, tₙ , t₂₇), until complete cessation of menstrual flow. In this manner the progression of OS over time was determined.

In the first 10 volunteers, the protocol also required that the E₂ level at times t₆, t₉, t₁₂, t₁₅, t₁₈, t₂₁ was evaluated, as well as the Pg levels at t₁₂ , t₁₈, t₂₁ in order to determine the correlation of OS with the secretory and luteal phases of the menstrual cycle.

E₂ and Pg were determined using known kits (Estradiol: Catalog No KE2D1; Progesterone: TKPG1; Inter Medico Markham, Ontario-Canada). The detection limits were, respectively, 5 pg/mL for E₂ and 0.1 ng/mL for Pg, with a coefficient of variation of 13.1% for E₂ and 6.5 % for Pg. Blood was collected from the brachial vein into heparinized test tubes (2 aliquots of 5 ml).

The general characteristics of the volunteers are given below:

| Characteristics | Mean values ± SD |
|---|---|
| Age (years) | 30.8 ± 5.66 |
| Weight (kg) | 63.2 ± 6.66 |
| Height (m) | 1.67 ± 0.07 |
| Body Mass Index (BMI kg/m²) | 22.6 ± 1.58 |

All the blood collections were carried out in the morning between 8 and 9.30, after fasting from the evening preceding the test, the analyses being determined on the same day. The volunteers were advised to avoid eating food to excess the day before the laboratory tests. The values for the d-ROMs test results and the E₂ and Pg levels are gathered together in Table 3.

**Table 3. Values for the d-ROMs test, E₂, Pg at various times in healthy volunteers (mean ± SD).**

| | | | | | | | | | | | |
|---|---|---|---|---|---|---|---|---|---|---|---|
| Cycle A control (no therapy) | Time days | t₁ | t₃ | t₆ | t₉ | t₁₂ | t₁₅ | t₁₈ | t₂₁ | t₂₄ | t₂₇ |
| | d-ROMs test U.CARR. | 284 ± 38.0 | 273 ± 47.0 | 284^{a} ± 34.6 | 299^{a} ± 31.3 | 336^{a} ± 66.8 | 378^{a} ± 115.6 | 326^{a} ± 66.4 | 315^{a} ± 56.3 | 323^{a} ± 51.1 | 276 ± 44.8 |
| | Estradiol pg/mL | | | 99 ± 33.7 | 222^{b} ± 34.2 | 195^{b} ± 39.9 | 134^{b} ± 36.2 | 157^{b} ± 36.9 | 126 ± 38.4 | | |
| | Progesterone ng/mL | | | | | 1.7 ± 0.54 | | 6.3^{c} ± 3.0 | 13.0^{c} ± 6.2 | | |
| ^{a} t-test for interdependent data, p< 0.05 tₙ Vs t₁; ^{b} t-test for interdependent data, p< 0.05 tₙ Vs t₆ ^{c} t-test for interdependent data, p<0.05 t₁₂ Vs tₙ | | | | | | | | | | | |

It was deduced from examining the data that OS arose between t₆ and t₂₄ and at t₂₇ the levels fell within the basal values at the start of the cycle. The concomitant maximum increase corresponded to a three to six day delay with respect to the estrogen peak.

The rise in Pg levels seemed to have the effect of reducing OS on attaining its peak in the blood (t₂₁) but not before.

It should be noted that, in terms of the d-ROMs test, the very OS is usually defined by values > 300 CARR. U. As deducible from the mean values, the increase in CARR. U. relative to the values at the start of the cycle reached a condition of OS from t₁₂ to t₂₄.

Correlations between E₂ and d-ROMs test levels were not observed; this indicated that the condition of increased systemic oxidation was not caused by E₂ alone but also other factors which, in the stated experimental conditions, were not able to be identified (referring to levels of LH, FSH, relaxin, etc.)

In any case, the unequivocal observation was confirmed that in the very estrogen phase, systemic oxidation values were found to rise, and then to fall with progesterone increase.

### Example 3.

### Evaluation of the activity of a contraceptive (CT) on OS levels and changes in OS following co-treatment with two different antioxidant product types

For this test 10 eumenorrheic women volunteers were selected, apparently healthy (a portion of those selected for Example 2), and not under any therapy. The general characteristics of the volunteers were the same as in Example 2.

For the entire test period, the volunteers attended the centre every three days in the mornings between 8.00 and 9.30, having fasted from the previous evening. Blood for the d-ROMs test evaluation was drawn from a finger and collected in an amount of 0.1-0.2 ml in microcuvettes containing heparin (10 µl of plasma sufficed for the d-ROMs test analysis).

The analyses were always carried out on the same day as the collection. Immediately after the blood collection, a standard breakfast was distributed. Otherwise, no other restrictions were imposed, apart from avoiding eating food to excess the evening before the collection.

The volunteers were monitored for the entire menstrual cycle and blood checks were carried out starting at the beginning of the menstrual cycle (t₁), then on day 3 (t₃) and then every three days until t₂₇ during the menstrual flow phase. The time period was identified as Cycle B.

The results are gathered together in Table 4.

**Table 4. d-ROMs test values at various times in healthy volunteers (mean ± SD)**

| | | | | | | | | | | | |
|---|---|---|---|---|---|---|---|---|---|---|---|
| Cycle B Control | Time in days | t₁ | t₃ | t₆ | t₉ | t₁₂ | t₁₅ | t₁₈ | t₂₁ | t₂₄ | t₂₇ |
| (no contraceptive therapy) | d-ROMs test U.CARR. | 248 ± 28.6 | 240 ± 26.5 | 255^{a} ± 26.8 | 284^{a} ± 22.8 | 276^{a} ± 21.3 | 271^{a} ± 25.9 | 267^{a} ± 26.1 | 256 ± 23.8 | 249 ± 32.2 | 246 ± 30.2 |

| | | | | | | | | | | | |
|---|---|---|---|---|---|---|---|---|---|---|---|
| *= t-test for interdependent data; tₙ Vs t₁ p< 0.05 in which n =6, 9, 12, 15, 18 | | | | | | | | | | | |

Even in this series of data, it could be seen that hydroperoxide levels (in terms of CARR. U.) as indices of oxidative state, increased from the blood check after 6 days (t₆) and remained as such until day t₁₈.

The data were slightly different from those obtained in Example 2 (the peak levels for the d-ROMs test were lower), but confirmed that OS arose during the estrogen peak at the ovum maturation phase, only to decrease during the very progesterone phase (combined estrogens and progesterones) until the subsequent menstrual flow occurred.

Also from these data it follows that during the estrogen phase, a condition of controlled OS was triggered i.e. there was an increase in hydroperoxide production, this being an index of increased oxidation, which sometimes exceeded the normal level of OS. Indeed, with the d-ROMs test, the condition of OS was determined as values > 300 CARR.U., which, under the present experimental conditions, were only found sporadically.

By analyzing individual cases, it could be seen that the highest increase in d-ROM test values was obtained between t₉ and t₁₅, i.e. upon the increase of E₂ (hence, generally slightly before the physiological peaks of FSH and LH; see Figure 1). The fact that the levels remained high until t₂₁ meant that the concomitant progesterone increase was unable to substantially change the oxidative condition, it tending to disappear only after the levels of both the hormones was reduced. The same volunteers then followed two 28-days cycles of hormonal contraceptive (CT) treatment with ethinylestradiol 50 µg combined with levonorgestrel 125 µg (Microgynon^{®}: 21 days of treatment and 7 inert).

During the second cycle (Cycle C) they were again subjected to serial testing for hydroperoxides, at the same times as the previous Cycles A and B, by using the same collection methods and identical health and hygiene protocol.

The results of the d-ROMs test are given in Table 5.

**Table 5. d-ROMs test values following treatment CT with Microgynon^{®} for 28 days in healthy volunteers (mean ± SD)**

| | | | | | | | | | | | |
|---|---|---|---|---|---|---|---|---|---|---|---|
| Cycle C (CT therapy) | Time days | t₁ | t₃ | t₆ | t₉ | t₁₂ | t₁₅ | t₁₈ | t₂₁ | t₂₄ | t₂₇ |
| | d-ROMS test U.CARR. | 241 ± 22.3 | 392* ± 26.3 | 398* ± 26.6 | 400* ± 24.4 | 409* ± 41.9 | 440* ± 35.3 | 434* ± 36.8 | 423* ± 33.9 | 290* ± 53.7 | 252 ± 28.3 |

| | | | | | | | | | | | |
|---|---|---|---|---|---|---|---|---|---|---|---|
| * = t-test for interdependent data p< 0.05 tₙ of Cycle A Vs tₙ of Cycle B | | | | | | | | | | | |

By examining the data a substantial increase in mean d-ROMs values was evident, being above normal values (aforenoted as <300 U.CARR.) which is evidence of the presence of OS starting even after the third day of CT therapy.

All the cases analyzed between t₃ and t₂₁ (time of CT pill consumption) were clearly above normal levels. Allowing for the fact the CT is substituted with an inactive pill on day t₂₁, it was logical that after three days of suspension (t₂₄) a recovery in the OS condition was detectable, and concluded at t₂₇. At t₂₇, none of the volunteers was actually in an OS condition.

If these data are compared with those of Cycle B (without CT therapy) it is clear that the values from t₃ to t₂₄ were significantly higher. This clearly showed that treatment with this hormonal contraceptive drug was a source of OS.

To the fourth consecutive treatment cycle (Cycle D), again based on ethinylestradiol 50 µg combined with levonorgestrel 125 µg (Microgynon^{®}: 21 treatment days and 7 inactive days), a daily administration was then added of a formulation having recognized and proven antioxidant activity, known as AR_{D} Stenovit ^{®} (AO_{St}).

This formulation comprised compounds with antioxidant activity, its formulation, as used in the present test, being given in Table 6.

In previous experiments, the same formulation was shown to possess an antioxidant action, determined on the basis of the d-ROMs test, when administered both to healthy volunteers [19] in a double-blind controlled experiment and in patients affected by peripheral arteriopathy [18].

**Table 6. Composition of the antioxidant formulation (AO_{St}).**

| Ingredients | Amount (mg) |
|---|---|
| Selenium yeast 0.2% | 24.00 |
| Protected Vitamin C 97.5% | 30.77 |
| Bioflavonoids from *Citrus* conc. 40% | 75.00 |
| Zinc pidolate | 25.00 |
| Coenzyme Q10 | 10.00 |
| L-cisteine hydrochloride | 12.61 |
| Vitamin E acetate conc. 50% | 32.91 |
| Pyridoxine hydrochloride | 1.22 |
| Vitamin A acetate 50000 IU/g | 0.7 |
| Beta-carotene 10% | 0.5 |

This antioxidant formulation (AO_{St}) in fact comprised all four types of antioxidant, i.e. membrane antioxidants (Vit E and beta-carotene), circulating antioxidants (Vit C L-bioflavonoids), cellular antioxidants (coenzyme Q and pyridoxine) and systemic antioxidants (selenium and L-cysteine) which are the components of GSH and GSHpx (respectively glutathione and glutathione peroxidase).

In the volunteers, AO_{St} was administered in the form of 10 ml biphasic vials (to be mixed at the point of administration) at the same time as the CT pill. The experimental methods of this cycle (Cycle D) were identical to those of the preceding Cycles B and C.

The results of the serial blood hydroperoxide values are given in Table 7.

**Table 7. d-ROMs test values following CT treatment (Microgynon^{®} for 28 days) combined with AO_{St} in healthy volunteers (mean ± SD).**

| | | | | | | | | | | | |
|---|---|---|---|---|---|---|---|---|---|---|---|
| Cycle D (CT therapy + AO_{St}) | Time days | t₁ | t₃ | t₆ | t₉ | t₁₂ | t₁₅ | t₁₈ | t₂₁ | t₂₄ | t₂₇ |
| | d-ROMS test U.CARR. | 241 22.3 | 391^{a} 21.2 | 395^{a} 20.0 | 398^{a} 19.0 | 407^{a} ± 39.5 | 438^{a} ± 36.9 | 427^{a} 36.7 | 423^{a} 33.7 | 310^{a} 80.4 | 257 36.1 |

| | | | | | | | | | | | |
|---|---|---|---|---|---|---|---|---|---|---|---|
| ^{a} p< 0.05 t-test for interdependent data t₁ Vs tₙ | | | | | | | | | | | |

As can be seen from the mean values at the various measurement times, treatment with AO_{St} which had proved to be able to reduce the d-ROMs test values in healthy volunteers of both sexes [19], was unexpectedly unable to act in any way on the OS deriving from the treatment with hormonal contraceptive drugs (CT).

Hence, it can be deduced from the results that OS originating from CT therapy was generated by rather particular mechanisms, such as to maintain it virtually unaltered despite administration of proven antioxidant formulations.

Following a subsequent wash-out cycle, wherein the volunteers continued only the treatment with the same CT, they were subjected to a further CT cycle (Cycle E) associated with the antioxidant composition of the invention (AOᵢₙᵥ), specifically given in Table 8.

**Table 8. Antioxidant composition of the invention (AOᵢₙᵥ)**

| | Amount (mg) |
|---|---|
| Selenium yeast* | 24.00 |
| Pyridoxine hydrochloride | 2.44 |
| Alpha-lipoic acid | 10.00 |
| Coenzyme Q10 | 10.00 |
| Beta-carotene 10% | 0.5 |
| Catechins, 40% being EGCG | 90.00 |

| | |
|---|---|
| * Selenium yeast titrated in organic selenium at 0.2% | |

The catechins were supplied in the form of green tea extract, wherein said catechins were 60% by weight.

The AOᵢₙᵥ composition was administered at the same time as the CT pill. Said composition was in the form of a kit, i.e. a powder and 10 ml of aqueous solution of decaffeinated green tea wherein the catechins were dissolved, to be mixed together at the time of consumption. The methods of collection and health and hygiene suggestions for this test were identical to those of the previous tests.

The results of the treatment with the composition of the invention are given in Table 9.

**Table 9. d-ROMS test values (mean ± SD) following combined treatment of CT (Microgynon^{®} for 28 days) and AOᵢₙᵥ for 27 days.**

| | | | | | | | | | | | |
|---|---|---|---|---|---|---|---|---|---|---|---|
| Cycle E +AO_{Inv}) | Time days | t₁ | t₃ | t₆ | t₉ | t₁₂ | t₁₅ | t₁₈ | t₂₁ | t₂₄ | t₂₇ |
| | d-ROMS test U.CARR. | 258 ± 18.4 | 352^{ab} ± 39.8 | 340^{ab} ± 34.1 | 318^{ab} ± 28.9 | 320^{ab} ± 34.2 | 314^{ab} ± 30.3 | 322^{ab} ± 30.8 | 329^{ab} ± 25.2 | 277^{ab} ± 15.0 | 244 ± 13.0 |

| | | | | | | | | | | | |
|---|---|---|---|---|---|---|---|---|---|---|---|
| ^{a} t-test for interdependent data p<0.05 tₙ Vs t₁ ^{b} t-test for interdependent data p< 0.05 tₙ of Table 8 Vs tₙ of Table 9 | | | | | | | | | | | |

With the exception of times t₁ and t₂₇, all the d-ROMs test values were significantly lower than those obtained with the contraceptive treatment alone; i.e. simultaneous treatment with the composition AOᵢₙᵥ was shown to be unexpectedly surprisingly and significant effective in reducing the OS during all the period of the cycle wherein the hormonal contraceptive drug was used.

These results were all even more surprising when considering that the use of the antioxidant product of proven and documented effectiveness, using the same type of test for determining the OS state, was found to be totally unsatisfactory.

### Example 4.

### Evaluation of the synergistic effect shown by the AOᵢₙᵥ compared with the action of catechins alone

For this test, 64 apparently healthy eumenorrheic volunteers were selected, all on contraceptive treatment for at least 6 months with a low dosage C containing ethinylestradiol 30 µg and drospirenone 3 mg (Yasmin^{®}), who were then subdivided into two groups: a control group and a treatment group.

The selection criteria for all the volunteers excluded concomitant chronic therapies, the pre-menopausal condition, the use of any types of food supplement, smoking and use of strong alcoholic drinks, excessive consumption of tea (>750 ml/day), of chocolate >50g/day) and a BMI > 25. The last pregnancy must have been no less than two years before the present test.

The control group was under a CT regimen associated with the controlled consumption of tea; the treatment group was under a regimen with the same CT with simultaneous consumption of the AOᵢₙᵥ composition as given in Table 6.

The control group (33 cases; mean age 35.9 ± 6.71 years) who took the CT according to the standard procedure suggested by the manufacturer, was subjected to analysis for monitoring of OS at three precise moments:
- a first determination for the selection, which had the aim of determining whether the CT had generated OS;
- a second determination to evaluate the basal value of OS;
- a third evaluation after 8 weeks of therapy with C.

As an integral part of the experimental protocol, said control group was required to consume two 150 ml cups of tea in the morning; one of them at breakfast and the other during the morning. In addition, it was requested that further tea was not consumed during the day.

The type of tea was identical for all volunteers in that it was supplied purposely for use in the experimental research (3 boxes each of 50 tea bags).

The catechins content in the tea [21, 23, 24] is given in Table 10.

**Table 10. Catechin content in the tea given out to the control group**

| Type of catechin | mg/cup | total mg/day |
|---|---|---|
| EGCG | 21.9 | 43.8 |
| EGC | 9.3 | 18.6 |
| ECG | 28.7 | 57.4 |
| EC | 10.0 | 20.0 |
| Total | 69.9 | 139.8 |

The request to consume at least two cups of tea derived from the choice to administer an amount of catechins that was definitely higher than that of the volunteers treated with the AOᵢₙᵥ composition. Administration of tea in the proposed amounts enabled the levels of catechins in blood and the relative antioxidant power [21] to be increased.

The volunteers of the treatment group (31 cases; average age 36 ± 8.22 years) followed the same scheme of CT therapy and OS monitoring, but after the basal check (after that of the selection) they were also given continuous treatment with AOᵢₙᵥ also for 8 consecutive weeks, after which OS was again checked. This group was asked to avoid tea consumption, or at the most to limit it to an amount not exceeding one cup/day (150 ml), as well as to avoid consuming chocolate for the 8 week research period.

The in vitro antioxidant action of the tea distributed to the volunteers of both groups was determined following an evaluation of AO power with the BAP method (biological antioxidant potency) [25]. In order to perform the test, a tea infusion was prepared by brewing a teabag for 5 minutes with 150 ml of boiling water. The vial of reconstituted AOᵢₙᵥ (powder dissolved in 10 ml of aqueous solution) was also brought to 150 ml with boiling water. The BAP was determined 10 minutes after their preparation. Six tea preparations, made at different times, were compared and assessed in duplicate with 6 AOᵢₙᵥ preparations also assessed in duplicate.

The in vitro results showed the antioxidant power of tea to be more efficient than the AOᵢₙᵥ composition; i.e. 3702 ± 347.3 µmol/l respectively of vitamin C for tea infusion and 3121 ± 136.8 µmol/l of vitamin C for AOᵢₙᵥ (p<0.05 t-test for independent data).

During the experimental period, all the volunteers were allowed to drink coffee during the day according to their custom. All the volunteers presented themselves for testing in the morning between 8.00 and 9.30, having fasted from the previous evening. They were advised to avoid eating food to excess the evening before the blood test which was carried out according to the methods given in Example 2. The scheme for evaluating OS consisted, as stated, of three controls; the control for selection, the baseline measurement, and the measurement after 8 weeks. The measurements were timed so as not to occur, for any of the cases, in the days immediately preceding the menstrual flow. The measurement results are given in Table 11.

As can be seen from the presented data, all the volunteers (both the control group and the treatment group), at the start of the observation period were in OS conditions. No volunteer actually had d-ROMs test values considered as being normal, i.e. < 300 Carr. U., as further confirmation that hormonal contraceptive drugs with low dosage estrogen also cause OS.

**Table 11. d-ROMs test values in healthy volunteers, before and after combined treatment of contraceptive (Yasmin®) and tea or and AOᵢₙᵥ, before and after an 8 week period (mean values ± SD)**

| Treatment | d-ROMs test values (U.CARR.) | |
|---|---|---|
| | basal | after 8 weeks |
| Control group [CT + tea] | 385 ± 38.2 | 394 ± 46.9 |
| Treatment group [CT + AOᵢₙᵥ] | 389 ± 44.2 | 305 ± 23.3 |

| | | |
|---|---|---|
| P< 0.05 t-test for interdependent data Controls Vs treated | | |

At the end of the 8 week period, it was noted that the d-ROMs test values for the group treated with AOᵢₙᵥ were significantly lower than the control group, with a high percentage of cases being within normal values (75%).

This did not happen in the control group, despite the volunteers having consumed a larger amount of catechins (with 98% compliance by counting the used tea bags).

Furthermore, the consumption of coffee in the two groups of the study was found to be practically the same: the average was 1.1 cups/day for the control group and 1.2 cups/day for the treatment group. Tea consumption by the volunteers of the group treated with AOᵢₙᵥ was averagely negligible (0.1 cups/day).

These results were surprising as they demonstrated that it was not the catechins which exerted an OS limiting action during the treatment regimen with the hormonal contraceptive drug CT, even at low dosages, since the catechins, though having been administered in higher amounts than those contained in the AOᵢₙᵥ, were found to be unable to act on OS.

It followed that the surprising results are ascribable to the antioxidant composition of the invention which, by suitably associating specific components with catechin action, resulted in an unexpected synergistic effect which enabled OS to be significantly reduced. These results were even more surprising and unexpected when considering that evaluation of antioxidant power in vitro, as reported above discouraged the use of the composition of the invention compared with green tea alone.

It should also be noted that this significant reduction advantageously leads to a consequently reduced frequency of superficial thrombotic episodes, as well as all those cardiovascular events attributable to the use of hormonal contraceptive drugs as a source of OS.

### Example 5.

### Evaluation of the synergistic effect shown by AOᵢₙᵥ with respect to the action of the components alone, i.e. without catechins

This test was conducted on 18 volunteers (a portion of the cases of Example 3). The aim of the test was to verify whether the antioxidants contained in the AOᵢₙᵥ, without green tea extract, were nevertheless effective in reducing the OS produced by the hormonal contraceptive drugs in use (Yasmin^{®}).

Therefore, three groups of volunteers were formed [A, B, C] each composed of 6 subjects selected on the basis of age, body weight and height such that all three groups were as homogeneous as possible. All the volunteers underwent a month of wash-out from every treatment except the contraceptive one. The characteristics of the volunteers are shown below:

| Characteristics* | Group A | Group B | Group C |
|---|---|---|---|
| | | | |
| Age (years) | 30.8 ± 3.43 | 32.7 ± 4.27 | 31.8 ± 5.81 |
| Weight (kg) | 63.3 ± 5.39 | 62.5 ± 2.66 | 63.3 ± 2.25 |
| Height (m) | 1.69 ± 0.06 | 1.68 ± 0.03 | 1.66 ± 0.04 |
| Body Mass Index BMI (kg/ m²) | 23.4 ± 1.40 | 22.1 ± 1.52 | 23.0 ± 1.30 |

| | | | |
|---|---|---|---|
| *All the general variables were analyzed by ANOVA and the groups were found to be not significantly different (p > 0.05). | | | |

The three volunteer groups were treated as follows:
- Group A was subjected to daily treatment with AOᵢₙᵥ;
- Group B was treated daily with green tea according to the methods given in Example 4 (consumption in the morning of two cups of tea, 150 ml each; again, one at breakfast and the other during the morning. In addition, it was requested to avoid consuming any other teas during the day);
- Group C was treated with the antioxidant composition given in Table 12, which corresponded to composition AOᵢₙᵥ without catechins. This last group was requested not to take tea throughout the duration of the test.

**Table 12. AO composition administered for 8 weeks to Group C**

| Ingredients | Amount (mg) |
|---|---|
| Selenium yeast conc. 0.2% | 24.00 |
| Pyridoxine hydrochloride | 2.44 |
| Alpha-lipoic acid | 10.00 |
| Coenzyme Q10 | 10.00 |
| Beta-carotene 10% | 0.5 |

The treatments were started with ongoing contraceptive therapy and were continued with no interruption for 8 weeks. During the test period, all the volunteers were allowed to drink coffee in the day time according to their custom. The Group C volunteers were also allowed to drink coffee if customary (provided it was not a replacement for tea).

Before starting taking the antioxidant formulations and for 8 weeks afterwards, the d-ROMs test values were acquired.

All the volunteers presented themselves for the blood checks between 8.00 and 9.30 in the morning, having fasted from the previous evening. They were advised to avoid eating food to excess the evening before the blood test which was carried out according to the methods given in Example 2.

The results are given in Table 13.

**Table 13. d-ROMs test values for the volunteers of Groups A, B and C, before and after treatment with antioxidant formulations. Mean values ± SD.**

| Period | Groups | | | ANOVA orthogonal contrasts |
|---|---|---|---|---|
| | A | B | C | |
| basal | 397 ± 9.9 | 389 ± 22.7 | 391 ± 13.5 | A=B=C |
| after 8 weeks | 315 ± 27.8 | 391 ± 29.2 | 401 ± 7.9 | B=C>A p<0.05 |

Examination of the data clearly showed that the treatment with formulations of antioxidants alone (Group C) or catechins alone (Group B, Example 4) were unable to affect OS at all. Therefore, it was shown that the significant effectiveness of composition AOᵢₙᵥ in reducing OS deriving from hormonal contraceptive drug treatment, was the result of combining catechins with suitable antioxidant components to give an unexpected and surprising synergistic effect.

### Bibliographical references

1) De Groote D, Perrier d'Hauterive S, Pintiaux A et al. Effect of oral contraception with ethinylestradiol and drospirenone on oxidative stress in women 18-35 years old. Contraception 2009;80:187-193.
2) Pincemail J, Vanbelle S, Gaspard U et al. Effect of different contraceptive methods on the oxidative status in women aged 40-48 years from ELAN study in the province of Liège- Belgium. Human Reprod 2007;22:2335-2343.
3) Loose D, Stancel G. Estrogens and progestins in: Goodman & Gilman's The pharmacological basis of therapeutics 11th Ed 2006:1541-1553.
4) Zachara BA, Gromadzińska J, W 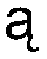 sowicz W, Zbróz. Red blood cell and plasma glutathione peroxidase activities and selenium concentration in patients with chronic kidney disease: a review. Acta Biochin Pol 2006;53:663-677.
5) Serviddio G, Loverro G, Vicino M et al. Modulation of endometrial balance during the menstrual cycle: relation with sex hormones. J Clin Endocrinol Metab 2002;87:2843-2848.
6) Massafra C, Gioia D, De Felice C et al. Effects of estrogens and androgens on erythrocyte antioxidant superoxide dismutase, catalase and glutathione peroxidase activities during the menstrual cycle. J Endocrinol 2000;167:447-452.
7) Santanan N, Shern-Brewer, McClatchey R et al. Estradiol as an antioxidant: incompatible with its physiological concentrations and function. J Lipid Res 1998;39:2111-2118.
8) Fukumura D, Gohongi T, Kadambi A et al. Predominant role of endothelial nitric oxide synthase in vascular endothelial growth factor-induced angiogenesis and vascular permeability. Proc Soc Acad Sci USA 2001;98:2604-2609.
9) Cameron IT, Campbell S. Nitric oxide in the endometrium. Human Repr Update 1998;4:565-589.
10) Bani D. Relaxin as a natural agent for vascular health. Vasc Health Risk Manag 2008;4:515-524.
11) Sugioka K, Shimosegawa Y, Nakano M. Estrogens as natural antioxidants of membrane phospholipid peroxidation. FEBS 1987;210:37-39.
12) Sack MN, Rader DJ, O Cannon R. Oestrogens and inhibition of oxidation of low-density lipoproteins in postmenopausal women. Lancet 1994;343:269-270.
13) Chiang K, Parthasarathy S, Santanan N. Estrogen, neutrophils oxidation. Life Sci 2004;75:2425-2438.
14) Larner JM, Pahuja SL, Shackleton CH et al. The isolation and characterization of estradiol-fatty acid esters in human ovarian follicular fluid. J Biol Chem 1993;268:13893-13899.
15) Tikkanen MJ, Vihma V, Jauhiainen M et al. Lipoprotein-associated estrogens. Cardiovasc Res 2001;56:184-188.
16) Larner JM, Shackleton CH, Roitman E et al. Measurement of estradiol-17-fatty acid esters in human tissue. J Clin Endocrinol Metab 1992;75:195-200.
17) Shern-Brewer R, Santanam N, Wetzstein C et al. Exercise and cardiovascular disease a new perspective. Arteriosc Thromb Vasc Biol 1998;18:1181-1187.
18) Cesarone MR, Belcaro G, Carratelli M et al. A simple test to monitor oxidative stress. Int Angiol 1999;18:127-130.
19) Cornelli U, Terranova R, Luca S, et al. Bioavailability and antioxidant activity of some food supplements in men and women using the D-Roms test as a marker of oxidative stress. J Nutr 2001;131:3208-3211.
20) Wolfram S. Effects of green tea and EGCG on cardiovascular and metabolic health. J Am Coll Nutr 2007;26:373S-388S.
21) Warden BA, Smith LS, Beecher GR et al. Catechins are bioavailable in men and women drinking black tea throughout the day. J Nutr 2001;131:1731-1737.
22) Saganuma M, Okabe S, Oniyama M et al. Wide distribution of [3H](-)epigallocatechin gallate, a cancer preventive tea polyphenol, in mouse tissue. Carcinogenesis 1998;19:1771-1776.
23) Schwedhelm E, Maas R, Troost R, Böger RH. Clinical pharmacokinetics of antioxidants and their impact on systemic oxidative stress. Clin Pharmacokinet 2003;42:437-459.
24) Fernandez-Panchon MS, Villano D, Troncoso AM, Garcia-Parrilla MC. Antioxidant activity of phenolic compounds: from in vitro results to in vivo evidence. Crit Rev Food Sci 2008;48:649-671.
25) Nakayama K, Terawaki H, Nakayama M et al. Reduction of serum antioxidative capacity during hemodialysis Clin Exp Nephrol 2007;11:218-225.

## Claims

1. Antioxidant composition comprising selenium yeast, vitamin B6, alpha-lipoic acid, coenzyme Q10, beta-carotene and catechins.

2. The antioxidant composition of claim 1, comprising 8-16% by weight of selenium yeast, 0.5-5% by weight of vitamin B6, 1-10% by weight of alpha-lipoic acid, 1-10% by weight of coenzyme Q10, 0.05-0.5% by weight of beta-carotene and 40-60% by weight of catechins.

3. The antioxidant composition of claim 2, comprising 10-14% by weight of selenium yeast, 1-3% by weight of vitamin B6, 3.5-6% by weight of alpha-lipoic acid, 3.5-6% by weight of coenzyme Q10, 0.1-0.3% by weight of beta-carotene and 42-51 % by weight of catechins.

4. The antioxidant composition of claim 3, comprising 12-13% by weight of selenium yeast, 1.1-2% by weight of vitamin B6, 4-5.5% by weight of alpha-lipoic acid, 4-5.5% by weight of coenzyme Q10, 0.2-0.3% by weight of beta-carotene and 45-48% by weight of catechins.

5. The antioxidant composition of any one of claims 1-4, wherein said catechins are epigallocatechin gallate (EGCG), epigallocatechin (EGC), epicatechin gallate (ECG) and epicatechin (EC).

6. The antioxidant composition of claim 5 wherein at least 35% by weight is EGCG on the catechin weight.

7. The antioxidant composition of any one of claims 1-6, wherein said catechins are in the form of green tea extract.

8. The antioxidant composition of any one of claims 1-7, wherein said vitamin B6 is pyridoxine or a salt thereof.

9. The antioxidant composition of any one of claims 1-8 for use as a medicament.

10. The antioxidant composition of any one of claims 1-8 for reducing oxidative stress ascribable to hormonal contraceptive drug treatment.

11. The antioxidant composition of claim 9 or 10, wherein one unit dose comprises 15.8-31.5 mg of selenium yeast, 1-9.5 mg of vitamin B6, 2-19 mg of alpha-lipoic acid, 2-19 mg of coenzyme Q10, 0.1-0.95 mg of beta-carotene and 80-110 mg of catechins.

12. The antioxidant composition of claim 11, wherein one unit dose comprises 20-27.5 mg of selenium yeast, 2-5.5 mg of vitamin B6, 7-11.8 mg of alpha-lipoic acid, 7-11.8 mg of coenzyme Q10, 0.2-0.59 mg of beta-carotene and 85-95 mg of catechins.

13. The antioxidant composition of claim 12, wherein one unit dose comprises 24 mg of selenium yeast, 2.44 mg of vitamin B6, 10 mg of alpha-lipoic acid, 10 mg of coenzyme Q10, 0.5 mg of beta-carotene and 90 mg of catechins, wherein about 40% is EGCG.

14. A kit comprising:
i) at least one composition comprising selenium yeast, vitamin B6, alpha-lipoic acid, coenzyme Q10 and beta-carotene; and
ii) at least one aqueous solution comprising catechins,
for the separate, sequential or simultaneous administration of the antioxidant composition of any one of claims 1-8.

15. The kit of claim 14, wherein said composition i) is in powder form.
